# EUROPEAN PATENT APPLICATION

(11) **EP 1 036 543 A1**
(43) Date of publication of application: **20.09.2000**
(21) Application number: 99307302.2
(22) Date of filing: 15.09.1999
(51) Int. Cl.: A61B 6/14

(54) **Data processing system, method and computer program product**

(30) Priority: 15.03.1999 KR 8508199
(71) Applicant: Samsung SDS Co. Ltd., Seoul (KR)
(72) Inventor: Lee, Yong-gu, 106-905 Life Apt., Sungnam-city, Kyungki-do (KR); Kim, Youngmin, 4-407 Kaepo Hanshin Apt., Seoul (KR)
(74) Representative: Hutchinson, Glenn Stanley

(57) **Abstract**

A data processing method and apparatus for visualising in 3 dimensions, the density of a jawbone at an implant region where an implant screw contacts the jawbone, using information about the density of the jawbone obtained by computed tomography (CT) and a modelled implant screw, and a computer readable medium therefor. The simulation method includes the steps of forming a 3-dimensional image of the jawbone, the image including information about the jawbone density. A colour map corresponding to the density distribution of the jawbone is set, and a modelled implant screw is modelled as a plurality of elements. Then, the modelled implant screw is inserted into a surgical region of the jawbone image, and values of the jawbone density at the points where each element of the modelled implant screw contacts the jawbone, are calculated. Then, colours are searched in the colour map corresponding to the density values of each element, and colouring faces of the modelled implant screw. Therefore, a dental surgeon can accurately ascertain the density of the jawbone at an implant region prior to actual implantation, thereby minimizing the side effects which may occur during actual implantation.

## Description

The present invention relates to a data processing system, method and computer program product and, more particularly, to a data processing system, method and computer program product for visualising in 3 dimensions, the density of a jawbone at the contact area between the jawbone and the surface of an implant screw, using a virtual implant screw and information about the density of the jawbone obtained through computed tomography (CT), and a computer readable medium therefor.

For the case where damage to teeth is too serious to repair, surgery for substituting artificial teeth for damaged teeth has become common. For such surgery, an implant screw for supporting the artificial teeth must be inserted into the jawbone.

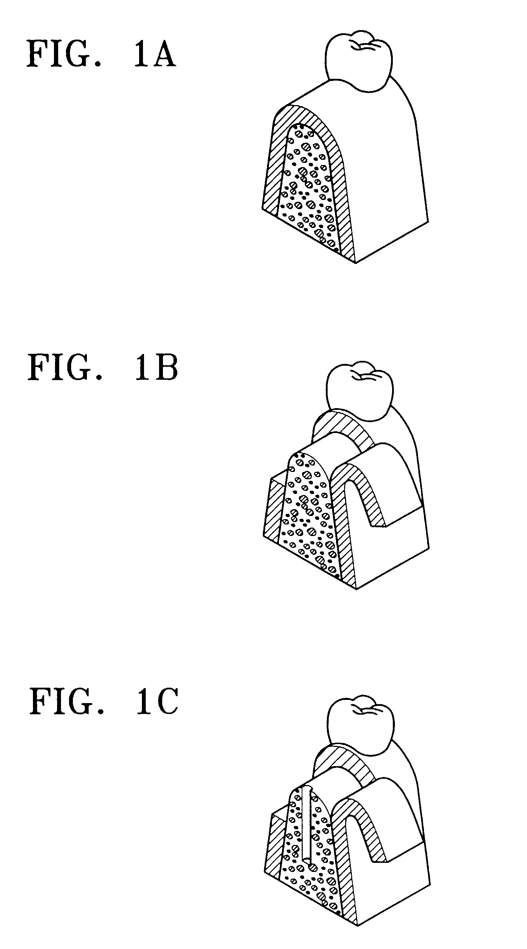

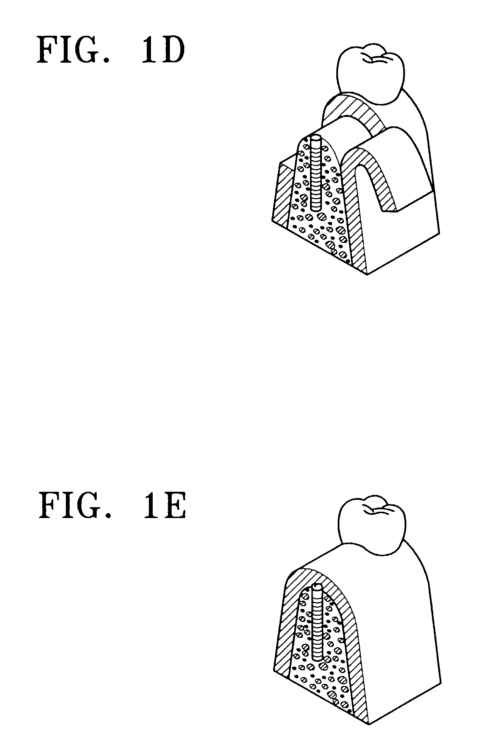

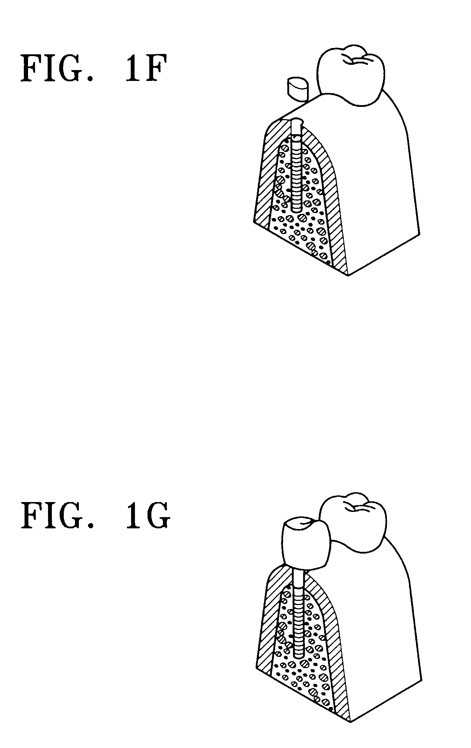

Figures 1A through 1G illustrate each step of implantation. In detail, in the case where a tooth is damaged due to damage as shown in figure 1A, an artificial tooth is implanted into the damaged region as follows. The gum in the damaged region is cut as shown in figure 1B, a region into which an implant screw is to be inserted is drilled to form a hole as shown in figure 1C, and the implant screw is inserted into the hole as shown in figure 1D. Then, the implanted region is left to allow the implant screw to bind firmly with the jawbone as shown in figure 1E. Usually, it takes about six months for the maxilla and about three months for the mandible. When the implant screw has firmly bound to the jawbone, the gum on the top of the implanted region is separated as shown in figure 1F and then an artificial tooth is mounted on the implant screw.

However, if a dentist fails to insert the implant screw into an appropriate region in an accurate direction during the above surgery, the implant screw cannot satisfactorily support the artificial tooth or the inappropriately inserted implant screw may encroach on alveolar nerves, causing facial paralysis. Thus, the most important step in implantation is to assess accurately the density of the jawbone in the vicinity of a desired implantation location. In particular, contact between the implant screw and a low-density area and particularly encroachment on the nerves in the jawbone should be avoided.

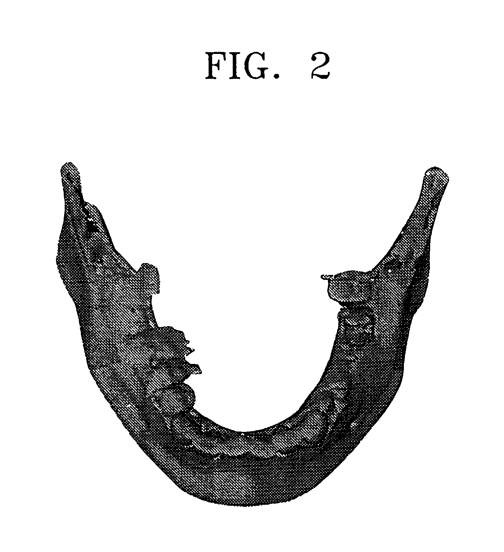

Success in implantation depends on how accurately a dental surgeon knows the jawbone quality of a patient. The current leading method in accurately ascertaining the jawbone quality is computed tomography (CT). CT refers to when an object is scanned in many directions and synthesized through computation, resulting in an image of intersection. At a dental surgery, during CT scanning, either the maxilla or the mandible is typically scanned in 1.0-mm increments, resulting in about 45 image slices. Figure 2 shows an image of a mandible obtained by CT.

As shown in figure 2, the images provided by CT are at intersections perpendicular to the cervical bone, which are not suitable for inspecting the contact area between the implant screw and the jawbone. This is why a reformatting technique is applied after the images have been scanned by CT. In general, the most prevalent reformatting orientation is perpendicular to the jawbone. However, because the jawbone is arch-shaped, the actual reformatted images are not parallel to each other. Also, images perpendicular to the jawbone as well as images perpendicular to the cervical bone are very helpful in examining an implant site. Unfortunately, images perpendicular to the jawbone do not show the density distribution of the jawbone in a 3-dimensional view, so that an implant surgeon cannot directly observe the density of jawbone at the contact area between the implant screw and the jawbone.

Due to the complicated 3-dimensional structure of the jawbone, it is difficult to observe accurately an implant site in the jawbone from only the intersection image perpendicular to the cervical bone. This is the reason why CT software for dentists provides a function capable of viewing the jawbone at different angles. For example, as the most common function of the CT software, a user can obtain a vertical sectional image by only drawing a parabola on a desired region of a given intersection, the vertical image corresponding to the desired region. However, using only the planar intersection images in identifying the density of a jawbone having a 3-dimensional structure is not suitable for precise implantation.

Accordingly, an aspect of the present invention, provides a data processing method for visualising the density of a jawbone at an implant region into which an implant screw for supporting an artificial tooth is inserted, comprising the steps of: (a) forming a 3-dimensional image of the jawbone, the image including information about the jawbone density; (b) setting a colour map corresponding to the density distribution of the jawbone; (c) modelling an implant screw as a plurality of elements; (d) inserting the modelled implant screw into a surgical region of the jawbone image; (e) calculating values of the jawbone density at the points where each element of the modelled implant screw contacts the jawbone; and (f) searching for colours in the colour map corresponding to the density values of each element, and colouring faces of the modelled implant screw.

Advantageously, embodiments of the present invention provide a simulation method for visualising the density of a jawbone in 3 dimensions, at the contact area between an implant screw and the jawbone, for implantation of the implant screw for supporting an artificial tooth in the jawbone, and a computer readable medium for the simulation method.

It will be appreciated that the implant region represents that region of the physical jawbone into which an implant screw is intended to be inserted whereas a surgical region represents that region of the 3-dimensional image of the jawbone, which corresponds to the implant region, into which the modelled implant screw is intended to be inserted.

The invention can also be embodied as computer readable code on a computer readable medium. The computer readable medium is any data storage device that can store data which can be thereafter be read by a computer system. Examples of the computer readable medium include read-only memory, random-access memory, CD-ROMs, magnetic tape, floppy disks, optical data storage devices. The computer readable medium can also be distributed over a network coupled computer systems so that the computer readable code is stored and executed in a distributed fashion.

According to another aspect of the present invention, there is provided a computer readable medium containing program instructions for visualising the density of a jawbone at an implant region into which an implant screw for supporting an artificial tooth is inserted, the computer readable medium comprising: computer readable code for forming a 3-dimensional image of the jawbone, the image including information about the jawbone density; computer readable code for setting a colour map corresponding to the density distribution of the jawbone; computer readable code for modelling an implant screw as a plurality of elements; computer readable code for inserting the modelled implant screw into a surgical region of the jawbone image; computer readable code for calculating values of the jawbone density at the points where each element of the modelled implant screw contacts the jawbone; and computer readable code for searching for colours in the colour map corresponding to the density values of each element, and colouring faces of the modelled implant screw.

According to a still further aspect of the invention there is provided a data processing apparatus for visualising the density of a jawbone at an implant region into which an implant screw for supporting an artificial tooth is inserted, comprising
means for forming a 3-dimensional image of the jawbone, the image including information about the jawbone density;
means for setting a colour map corresponding to the density distribution of the jawbone;
means for modelling the implant screw on a plurality of elements;
means for inserting the modelled implant screw into a surgical region of the jawbone image;
means for calculating values of the jawbone density at the points where each element of the modelled implant screw contacts the jawbone; and
means for searching for colours in the colour map corresponding to the density values of each element, and colouring faces of the modelled implant screw.

Embodiments of the present invention will now be described by way of example only with reference to the attached drawings in which:
Figures 1A through 1G illustrate each step of dental implantation;
Figure 2 shows an image of a mandible obtained by computed tomography (CT);
Figure 3 is a flowchart of a data processing method for visualising the density of a jawbone according to an embodiment of the present invention;
Figure 4 shows a modelled implant screw used to identify the bone density at an implant area in an embodiment of the present invention;
Figures 5A, 5B and 5C show basic shapes of elements consisting of the head, the rod and the end tip of the modelled implant screw shown in figure 4;
Figure 6 illustrates a state where the modelled implant screw implant is inserted into a surgical region of a mandible;
Figures 7A, 7B and 7C illustrate the calculation of CT number at a node of a element by the grid points of CT slices; and
Figure 8 shows a modelled implant screw which is coloured according to the CT numbers.

Computed tomography (CT) which is essential for diagnosing the internal status of a damaged part without surgery, gives CT numbers of a continuous 3-dimensional object at a finite number of rectilinear grid points. Also, because the CT number is directly proportional to the density of the object at the corresponding point, the CT numbers are interchangeable with the density of the object.

In a simulation method according to an embodiment of the present invention, the density of a jawbone at an implant region can be visualised in 3 dimensions from the information about the bone density obtained by the CT, using a modelled implant screw, which is modelled to have a similar shape to that of an actual implant screw, inserted into the implant region.

The main idea for such visualisation is in positioning the modelled implant screw into an image obtained from CT slices, and colouring the surface of the screw according to density values. Here, colouring is performing by a predetermined colour map which has been set corresponding to each CT number. Here, the simulation method can be simplified by modelling the implant screw as elements and then colouring the elements. The details are as follows: (1) the modelled implant screw is modelled as a mesh consisting of elements, (2) the modelled implant screw is positioned at a surgical region of the image obtained by CT, by the necessary translation and rotation, (3) density values (CT numbers) corresponding to each node of the elements are computed by an interpolation method, such as a linear interpolation method, and (4) parts, preferably faces, of elements, i.e., the surface of the modelled implant screw, are coloured according to node values using a colour map.

According to an embodiment of the present invention, a 3-dimensional jawbone model is reconstructed from CT images of a patient and virtual implantation is tried on the 3-dimensional jawbone model, such that the side effect of real implantation, which depends on the location and orientation of a surgical region, can be inspected or determined in advance. The 3-dimensional restructuring of the jawbone and virtual implantation will now be described.

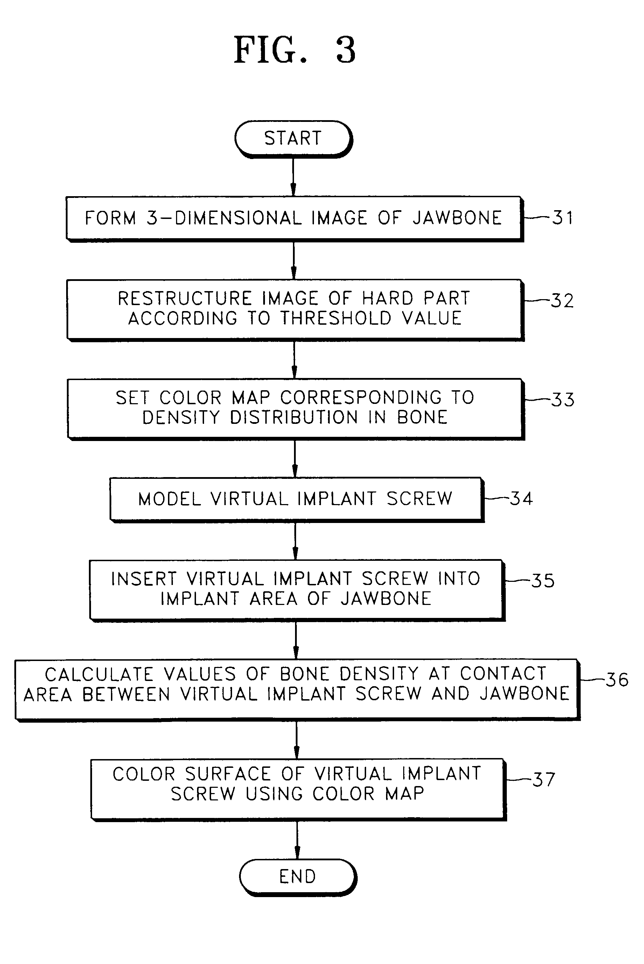

Figure 3 is a flowchart illustrating a simulation method for visualising the density of a jawbone according to an embodiment of the present invention.

A plurality of CT slices for a jawbone are obtained in a direction perpendicular to the cervical bone, and a 3-dimensional CT image of the jawbone, as shown in figure 2, is obtained using the CT slices. The CT number of each pixel in the CT slice ranges from 0 to(*2*^{*N*}*-1*) (where N is the number of bits used to represent each pixel) . If the number of bits is 16, the maximum CT number becomes 65,535. The jawbone image can be restored into a 3-dimensional structure or at an arbitrary angle by stacking the CT slices and is also reformed into an oblique cross-sectional image therefrom (step 31).

CT refers to a method in which X-rays are irradiated around an object at a predetermined angle and the density at each point inside the object is calculated using the resulting plurality of images which have been obtained by X-ray irradiation. In general, the X-rays are absorbed better in a high-density region than in a low-density region. A region of a CT image indicated by a relatively large CT number represents a high-density region such as bone, a region of a CT image indicated by a relatively small CT number represents a low-density region such as space, and a region indicated by an intermediate CT number represents a soft part (tissue) of the human body. Here, as the CT number becomes larger, the image is displayed in a brighter colour on a cathode ray tube (CRT). The region of interest in the present invention is the hard part, so that only the image of the hard part is separated to reform its 3-dimensional image. However, the hard part is not clearly distinct from the soft part. That is, there is a transition region between the hard and soft parts. A "threshold value" is used as a value for distinguishing the hard part from the soft part, and all CT numbers smaller than the threshold value are set to zero or to a minimum value. In this way, the image of only the hard part is separated from the entire CT image and a 3-dimensional image of the jawbone can be reconstructed (step 32). Also, a user can appropriately select a threshold value by inspecting how the 3-dimensional image of the hard part changes according to threshold values applied to the CT image.

In general, the CT slices are often annotated with various computer-added texts, for example, the slice number. However, such information can be removed during the step 32. In detail, because the intensity of these annotations is constant and is greater than that of the background intensity, the algorithm searches for pixels in the image having the largest intensity and then sets the intensity of all searched pixels to zero or the minimum value. In this way, the useless information can be removed.

The boundary between the hard part and the soft part can be distinguished by extracting CT numbers having a threshold value from a set of CT numbers for all slices. Here, the following operation may be performed in order to increase the processing speed of a computer.

When CT slices are stacked, a box-shaped image is obtained. Here, a small box region in which a user is interested, can be defined, rather than performing computation for the entire image, which is called "data regioning". Also, the hard part of the jawbone may be displayed as separate anatomical parts or as a whole image by lowering the threshold value. Here, each piece of the hard part, which is separated from another, is called a "lump". In an embodiment of the present invention, only a required lump is selected and the selected lump is three-dimensionally reformed. Also, the reforming of lumps can be performed two-dimensionally, rather than three-dimensionally, in order to reduce the amount of computation. That is, computation for 26 neighboring pixels in 3 dimensions is simplified into the computation for 8 neighboring pixels in 2 dimensions. According to the simplified computation, the values of pixels located at the same position in each slice (pixels located on the same vertical axis when the CT slices are stacked) are summed, the image of a representative one CT slice is computed, and the computation of 8 neighboring pixels in 2 dimensions is performed. Here, the 26 neighboring pixels include the pixels located around the faces, the edges and the vertices with respect to a pixel, and the 8 neighboring pixels include the pixels located around the edges and vertices with respect to a pixel.

To simplify data processing, the following process may be performed. After analyzing the connectivity of the jawbone from the 26 neighboring pixels in 3 dimensions, lumps other than the largest lump may be erased, or an image of an artificial element in the oral cavity, such as a metal filling which causes interference to the CT image, may be automatically corrected to remove a flash. Also, in order to improve the display speed of a screen during real-time rendering, it is desirable to reduce the number of triangles the user intends to draw during the rendering. In particular, most triangles placed on the same plane are combined, resulting in a small number of triangles. Methods for reducing the number of triangles includes vertex-based decimation and edge-based decimation. In vertex-based decimation, triangles that share vertices are erased, so that a small number of large triangles are drawn in the resulting space. Meanwhile, in edge-based decimation, two triangles that share an edge are combined into one large triangle by erasing the shared edge.

Afterwards, a colour map corresponding to the CT numbers, that is, the density distribution of the jawbone, is prepared (step 33). Blue, green and red are used; blue is assigned to the maximum CT number, red is assigned to the minimum CT number, and green is assigned to the CT number corresponding to the arithmetic average of the maximum and minimum CT numbers, resulting in two spans: one between blue and green, and the other between green and red. If a CT number is given, the span corresponding to the CT number is determined, and the colour of the CT number is determined by linearly interpolating two end colours of the corresponding span. By doing so, the density of the jawbone at an implant area where the virtual implant screw contacts the jawbone can be visualised in colour as a 3-dimensional image.

Alternatively, the density distribution of the jawbone may be displayed in a gray scale. The maximum density is set as black and the minimum density is set as white, and then linear interpolation is performed using the maximum and minimum density values such that a given density is displayed in a gray scale.

In the case where the CT number for each pixel of a CT is expressed with 16 bits, the value which a CT number can take is one of 65,536 numbers. Meanwhile, a general computer monitor can display only 256 brightness values in gray levels. Thus, displaying 65,536 CT numbers with 256 colours lowers discrimination of parts of the image. Even though 256 colours are not sufficient to display the CT images corresponding to all CT numbers, the distribution of CT numbers can be effectively expressed with 256 colours by delimiting only an area of interest in the hard part.

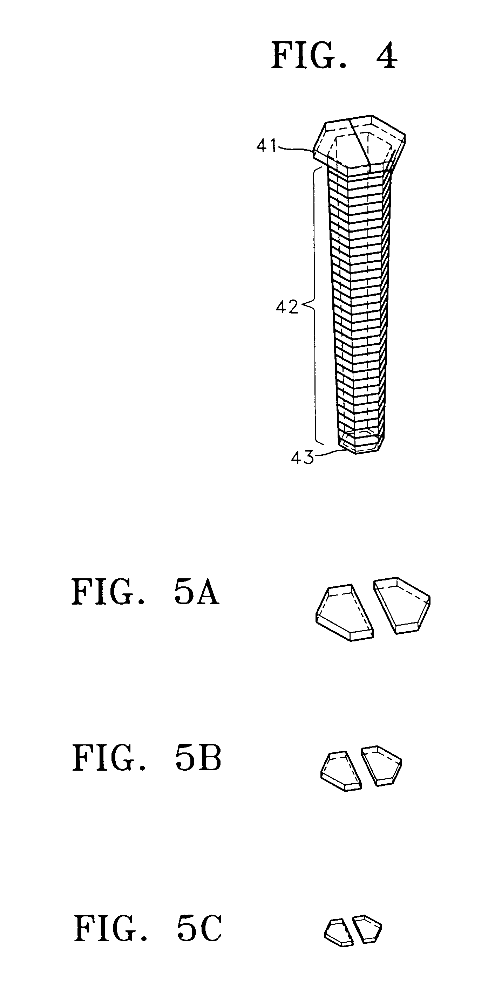

The implant screw is modelled as elements in step 34. Figure 4 shows an implant screw model used in order to identify the density of the jawbone at an implant area according to an embodiment of the present invention. Figures 5A, 5B and 5C show the basic shapes of the head, the rod and the end tip of the implant screw, respectively, as the elements of the implant screw model.

The modelled implant screw shown in figure 4 comprises three parts: a head 41, a rod 42 and an end tip 43, and is formed by sequentially stacking elements such as those shown in FIGURES. 5A, 5B and 5C. The head 41 is modelled as a hexagon having a cross-section which is larger than that of the rod 42, the rod 42 has a similar shape to the head 41, but is longer than the head 41, and the end tip 43 is also modelled as a hexagon having tapered sides. Here, the modelled implant screw is modelled to a shape which is nearly the same as that of a typical implant screw used in actual implantation. However, the modelled implant screw may be modelled in more detail if required. Also, modelling of the implant screw depends on the resolution of a CT scanner, so that it is impractical to model a virtual implant screw more precisely than the resolution of the CT scanner.

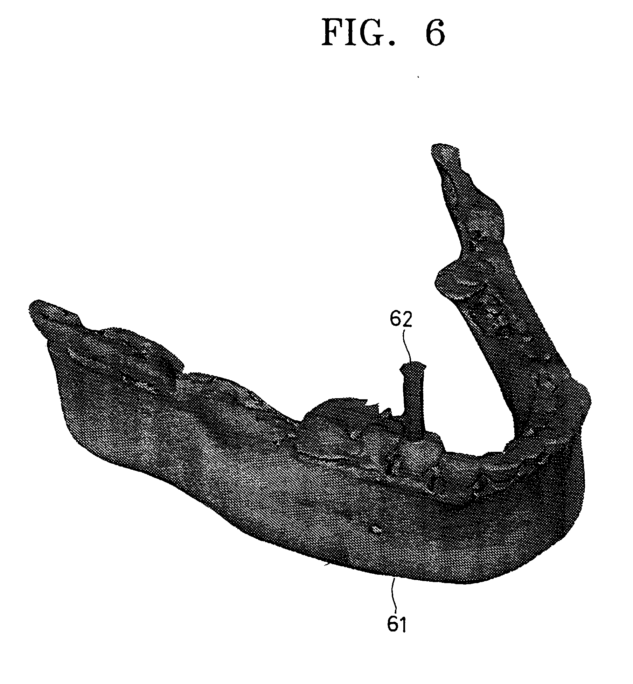

In step 35, the implant screw model is inserted into an implant area of a jawbone. Figure 6 shows the state where the modelled implant screw 62 is inserted into an surgical region of the image of the jawbone 61 obtained through CT. A dental surgeon can arbitrarily determine the location, orientation and depth of the modelled implant screw to be inserted.

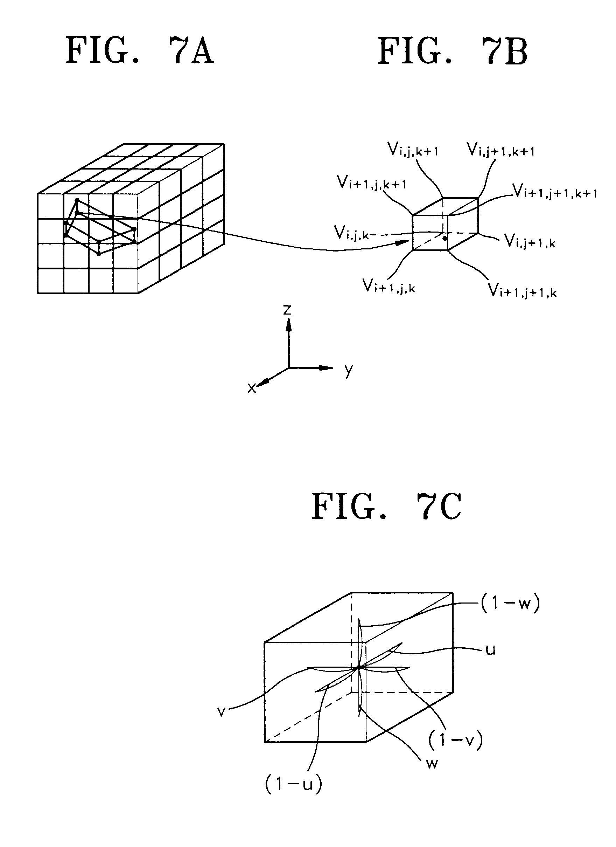

In step 36, the CT numbers corresponding to nodes of each element of the modelled implant screw inserted into the CT image of the jawbone are computed. Figures 7A through 7C illustrate the calculation of CT numbers corresponding to each node of the element in relation to the grid points of CT slices. In general, the CT numbers are only defined on the grid points of the CT slices and the nodes of the element do not necessarily coincide with the grid points. Thus, the CT value of a node may be calculated by interpolating CT numbers at the grid points. Assuming that a node exists in a hexahedron as shown in FIGURE. 7B, the location of the node is expressed as a relative position with respect to the neighboring grid points shown in FIGURE. 7C, using u, v and w, which are distances from the node to the faces of the grid in x, y and z-axis directions. The value *V*_{*N*} of the node is calculated by the following mathematical relation (1).${\text{V}}_{\text{N}} \text{= (1 -} \text{u} \text{)(1 -} \text{v} \text{)(1 -} \text{w} \text{)} {\text{V}}_{\text{i,j,k}} \text{+ (1 -} \text{u} \text{)} \text{v} \text{(1 -} \text{w} \text{)} {\text{V}}_{\text{i,j}} {\text{}}_{\text{+1}} {\text{}}_{\text{,k}} \text{+} \text{u} \text{(1 -} \text{u} \text{)(1 -} \text{w} \text{)} {\text{V}}_{\text{i}} {\text{}}_{\text{+1}} {\text{}}_{\text{,j,k}} \text{+} \text{uv} \text{(1 -} \text{w} \text{)} {\text{V}}_{\text{i}} {\text{}}_{\text{+1,j+1}} {\text{}}_{\text{,k}} \text{+ (1 -} \text{u} \text{)(1 -} \text{v} \text{)} {\text{wV}}_{\text{i,j,k}} {\text{}}_{\text{+1}} \text{+ (1-} \text{u} \text{)} {\text{vwV}}_{\text{i,j}} {\text{}}_{\text{+1,} \text{k} \text{+1}} \text{+} \text{u} \text{(1 -} \text{v} \text{)} {\text{wV}}_{\text{i}} {\text{}}_{\text{+1,}} {\text{}}_{\text{j}} {\text{}}_{\text{+1,}} {\text{}}_{\text{k}} {\text{}}_{\text{+1}} \text{+} {\text{uvwV}}_{\text{i}} {\text{}}_{\text{+1,}} {\text{}}_{\text{j}} {\text{}}_{\text{+1,}} {\text{}}_{\text{k}} {\text{}}_{\text{+1}}$

After the CT values of eight nodes of the element have been calculated, colours corresponding to the values are selected from a colour map table, and the sides of the element are coloured.

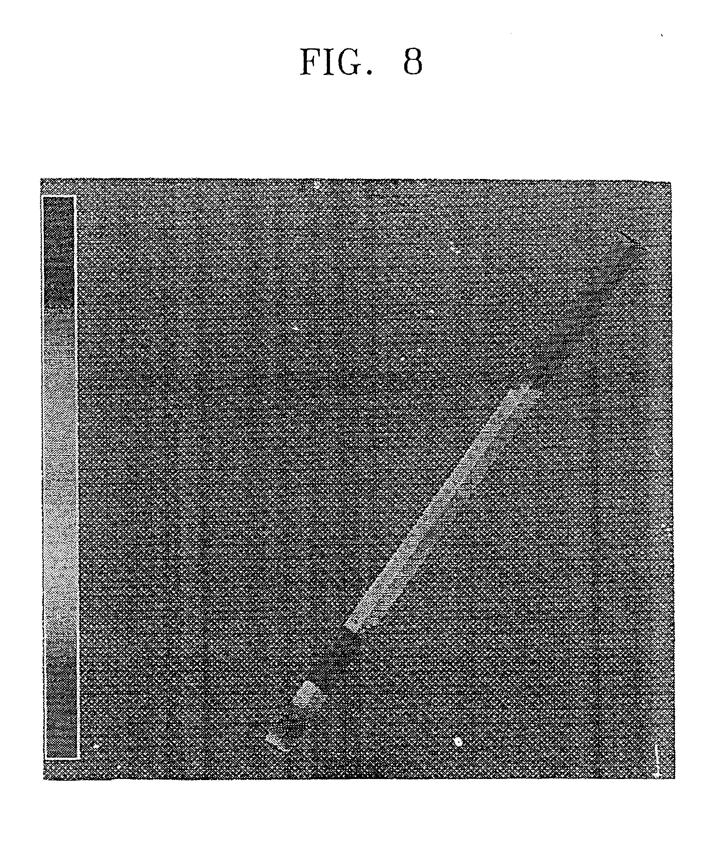

In step 37, the entire modelled implant screw is coloured according to the CT values of each node of the elements with reference to the colour map table. That is, the sides of each element contained in the implant screw are coloured in different colours according to the CT values. Figure 8 shows the modelled implant screw having elements which are coloured according to the density of the jawbone. The bar located at the left of figure 8 represents the relationship between the bone density and colours, and the coloured modelled implant screw located at the right represents the simulation result. In figure 8, the bar can be a gray scale image. However, the bar may be displayed with real colours, using red, green and blue in an actual application.

As shown in figure 8, the density distribution of a jawbone at the implant area where the modelled implant screw and the jawbone contact can be visualised by colouring the surface of the modelled implant screw, according to the location, orientation and depth of the implant screw. Thus, it can be determined whether the density of a jawbone is high enough for implantation. In particular, a high density of a jawbone around the implant area is important for strong binding of the implant screw with a jawbone.

The invention can also be embodied as computer readable code on a computer readable medium. The computer readable medium is any data storage device that can store data which can be thereafter be read by a computer system. Examples of the computer readable medium include read-only memory, random-access memory, CD-ROMs, magnetic tape, floppy disks, optical data storage devices. The computer readable medium can also be distributed over a network coupled computer systems so that the computer readable code is stored and executed in a distributed fashion.

As described above, in the simulation method for visualising the density of a jawbone according to the present invention, an implant screw modelled as elements is inserted into the image of a jawbone, obtained by CT, and the bone density at the area where the modelled implant screw contacts the jawbone is calculated. Then, the modelled implant screw is coloured with different colours, according to the bone density, thereby visualising the density of the jawbone at the implant area in 3 dimensions. Accordingly, a dental surgeon can accurately ascertain the density of jawbone at an implant area, thereby minimizing possible side effects in actual implantation.

## Claims

1. A data processing method for visualising the density of a jawbone at an implant region into which an implant screw for supporting an artificial tooth is inserted, comprising the steps of:
(a) forming a 3-dimensional image of the jawbone, the image including information about the jawbone density;
(b) setting a colour map corresponding to the density distribution of the jawbone;
(c) modelling the implant screw as a plurality of elements;
(d) inserting the modelled implant screw into a surgical area of the jawbone image;
(e) calculating values of the jawbone density at the points where each element of the modelled implant screw contacts the jawbone; and
(f) searching for colours in the colour map corresponding to the density values of each element, and colouring parts of the modelled implant screw.

2. The data processing method of claim 1, wherein in the step (c) of modelling the implant screw, the modelled implant screw comprises a head modelled as a protruded hexagonal shape, a rod modelled as a hexagonal shape to be longer than the head and attached underneath the head, and an end tip modelled as a hexagonal shape having tapered sides.

3. The data processing method of either claims 1 and 2, after the step (a) of forming the image of the jawbone, further comprising the step of reforming the image of the jawbone by extracting an image of a hard part having a density higher than a predetermined threshold value.

4. The data processing method of any preceding claim, wherein in the step (b) of setting the colour map, blue is assigned to the maximum value of bone density, red is assigned to the minimum value of bone density, and green is assigned to the value of bone density corresponding to the arithmetic average of the maximum and minimum values of bone density; two spans are formed, one between blue and green and the other between green and red; and the colour of a specific value of bone density is determined by linearly interpolating two end colours of the corresponding span.

5. The data processing method of any preceding claim, wherein in the step (b) of setting the colour map, the colour map is set as a gray scale varying from the maximum value of bone density to the minimum value of bone density.

6. A data processing apparatus for visualising the density of a jawbone at an implant region into which an implant screw for supporting an artificial tooth is inserted, comprising
means for forming a 3-dimensional image of the jawbone, the image including information about the jawbone density;
means for setting a colour map corresponding to the density distribution of the jawbone;
means for modelling the implant screw on a plurality of elements;
means for inserting the modelled implant screw into a surgical region of the jawbone image;
means for calculating values of the jawbone density at the points where each element of the modelled implant screw contacts the jawbone; and
means for searching for colours in the colour map corresponding to the density values of each element, and colouring faces of the modelled implant screw.

7. The data processing apparatus of claim 6, wherein in the means for modelling the implant screw, the modelled implant screw comprises a head modelled as a protruded hexagonal shape, a rod modelled as a hexagonal shape to be longer than the head and attached underneath the head, and an end tip modelled as a hexagonal shape having tapered sides.

8. The data processing apparatus of either of claims 6 and 7, further comprising means for reforming the image of the jawbone by extracting an image of a hard part having a density higher than a predetermined threshold value.

9. The data processing apparatus of any of claims 6 to 8, wherein in the means for setting the colour map, blue is assigned to the maximum value of bone density, red is assigned to the minimum value of bone density, and green is assigned to the value of bone density corresponding to the arithmetic average of the maximum and minimum values of bone density; two spans are formed, one between blue and green and the other between green and red; and the colour of a specific value of bone density is determined by linearly interpolating two end colours of the corresponding span.

10. The data processing apparatus of any of claims 6 to 9, wherein in the means for setting the colour map, the colour map is set as a gray scale varying from the maximum value of bone density to the minimum value of bone density.

11. A computer program product comprising a computer readable storage medium having stored thereon computer program code means for implementing a method of any of claims 1 to 6 or a data processing system of any of claims 6 to 10.

12. A computer program product for visualising the density of a jawbone at an implant region into which an implant screw for supporting an artificial tooth is inserted, the computer program product comprising a computer readable medium having:
computer readable code for forming a 3-dimensional image of the jawbone, the image including information about the jawbone density;
computer readable code for setting a colour map corresponding to the density distribution of the jawbone;
computer readable code for modelling the implant screw as a plurality of elements;
computer readable code for inserting the modelled implant screw into a surgical region of the jawbone image;
computer readable code for calculating values of the jawbone density at the points where each element of the modelled implant screw contacts the jawbone; and
computer readable code for searching for colours in the colour map corresponding to the density values of each element, and colouring faces of the modelled implant screw.
